# EUROPÄISCHE PATENTANMELDUNG

(11) **EP 1 721 627 A1**
(43) Veröffentlichungstag der Anmeldung: **15.11.2006**
(21) Anmeldenummer: 06008031.4
(22) Anmeldetag: 18.04.2006
(51) Int. Cl.: A61M 11/06

(54) **Inhalationstherapievorrichtung mit Verneblerdüse und Kompressor**

(30) Priorität: 13.05.2005 DE 102005022339
(71) Anmelder: PARI GmbH Spezialisten für effektive Inhalation, 82319 Starnberg (DE)
(72) Erfinder: Boehm, Andreas, 86934 Reichling (DE); Kunschir, Eduard, 80469 München (DE)
(74) Vertreter: HOFFMANN EITLE

(57) **Zusammenfassung**

Die Inhalationstherapievorrichtung umfasst eine Verneblerdüse 1, der für die Vernebelung einer Flüssigkeit 5 Druckluft 6 zuführbar ist, einen Kompressor 8, der einen elektromotorischen Antrieb 8b aufweist, für die Erzeugung von der Verneblerdüse zugeführter Druckluft, und einen elektrischen Energiespeicher 9 für die Bereitstellung von elektrischer Energie für den elektromotorischen Antrieb 8b des Kompressors 8. Dabei weist der elektrische Energiespeicher 9 eine Konstanthalteeinrichtung 9b auf, die eine konstante Ausgangsspannung des Energiespeichers 9 gewährleistet.

## Beschreibung

Die Erfindung betrifft Inhalationstherapievorrichtungen mit einer Verneblerdüse für die Vernebelung einer Flüssigkeit mit Hilfe von Druckluft und mit einem Kompressor für die Bereitstellung von der Verneblerdüse zuzuführender Druckluft.

Für die Erzeugung eines Aerosols zur Durchführung einer Inhalationstherapie haben sich neben anderen Primäraerosolerzeugern in besonderem Maße Verneblerdüsen bewährt, die mit Hilfe von zugeführter Druckluft aus einer Flüssigkeit ein Aerosol erzeugen. Verneblerdüsen sind beispielsweise aus DE 27 11 060 A, DE 89 05 364 U oder DE 196 02 628 A bekannt. Die Druckluft für die Aerosolerzeugung wird mit Hilfe eines Kompressors bereitgestellt, der in der Regel einen Elektromotor als Antrieb aufweist. Bei mobilen Inhalationstherapiegeräten, wie beispielsweise in DE 94 18 334 U beschrieben, wird der Kompressor mit Hilfe eines Akkumulators als elektrischem Energiespeicher betrieben.

Im Stand der Technik sind verschiedene wiederaufladbare elektrische Energiespeicher bekannt, die sich für den Einsatz in einem mobilen Inhalationstherapiegerät anbieten. Im Unterschied zu elektrischen Energiespeichern für elektronische Geräte, beispielsweise im Bereich der mobilen Informations- und Kommunikationssysteme, ist bei mobilen Inhalationstherapiegeräten mit einer Verneblerdüse nicht davon auszugehen, dass für den Betrieb des Kompressors ein elektrischer Energiespeicher mit besonderen Eigenschaften eingesetzt werden sollte. Im Grunde eignen sich eine Vielzahl von Akkumulatorsystemen für diesen Anwendungsfall. Zwar ist im Stand der Technik bekannt, im Bereich der mobilen Informations- und Kommunikationssysteme elektrische Energiespeiche einzusetzen, bei denen mit Hilfe eines Reglers eine konstante Ausgangsspannung gewährleistet wird, wie beispielsweise DE 101 38 515 A beschreibt. Jedoch unterscheidet sich davon das Anwendungsumfeld bei Inhalationstherapiegeräten mit Verneblerdüse und Kompressor erheblich, da der Kompressor als ein elektrisches Aggregat angesehen werden muss, das nur sehr geringe Ansprüche an die Spannungsversorgung stellt, ganz anders als beispielsweise ein Gerät aus der mobilen Informations- und Kommunikationstechnik.

Vor diesem Hintergrund schlägt die vorliegende Erfindung vor, ein Inhalationstherapiegerät mit Verneblerdüse und Kompressor auszustatten mit einem elektrischen Energiespeicher (Akkumulator/Akkupack), bei dem mit Hilfe eines Reglers oder Spannungswandlers, beispielsweise eines DC/DC-Wandlers eine konstante Versorgungsspannung für den Betrieb des Kompressors bereitgestellt wird.

Ein Inhalationstherapiegerät gemäß der Erfindung ist nicht nur im Hinblick auf die elektrischen Komponenten, also insbesondere den Elektromotor des Kompressors vorteilhaft, sondern entfaltet auch eine vorteilhafte Wirkung im Hinblick auf die mit dem erfindungsgemäßen Inhalationstherapiegerät durchgeführte Inhalationstherapie. Denn durch die Bereitstellung einer konstanten Versorgungsspannung für den Betrieb des Kompressors eines mobilen Inhalationstherapiegeräts wird gewährleistet, dass das Gesamtsystem hinsichtlich der Abgabemenge (TOR = total output rate) und der Qualität (MMD = mass median diameter) des Aerosols verbessert ist und gleichmäßigere Werte erzielt werden. Damit erhöht sich die Dosisgenauigkeit, die mit einem erfindungsgemäßen Inhalationstherapiegerät erzielt werden kann.

Eine erfindungsgemäße Inhalationstherapievorrichtung umfasst eine Verneblerdüse, der für die Vernebelung einer Flüssigkeit Druckluft zuführbar ist, einen Kompressor, der einen elektromotorischen Antrieb aufweist, für die Erzeugung von der Verneblerdüse zugeführter Druckluft, und einen elektrischen Energiespeicher für die Bereitstellung von elektrischer Energie für den elektromotorischen Antrieb des Kompressors. Dabei weist der elektrische Energiespeicher eine Konstanthalteeinrichtung auf, die eine konstante Ausgangsspannung des Energiespeichers gewährleistet. Damit steht für das Inhalationstherapiegerät ein Akku zur Verfügung, der eine konstante Ausgangsspannung und somit eine definierte maximale Leistung liefert.

Die Konstanthaltungseinrichtung kann beispielsweise in Form eines DC/DC-Wandlers realisiert sein. Dadurch ist eine Gefährdung des Akkus durch Kurzschluss oder durch Tiefentladung praktisch ausgeschlossen.

In einer vorteilhaften Ausgestaltung weist der elektrische Energiespeicher ein Gehäuse auf, in dem die Konstanthaltungseinrichtung angeordnet ist. Damit steht ein einheitlich handhabbarer Akku zur Verfügung.

Um ein kompaktes Gesamtsystem zu erzielen, ist das Gehäuse des elektrischen Energiespeichers in einer vorteilhaften Gestaltung in Form und Größe an das Gehäuse des Kompressors angepasst.

Die Erfindung wird im Folgenden anhand eines Ausführungsbeispiels beschrieben.

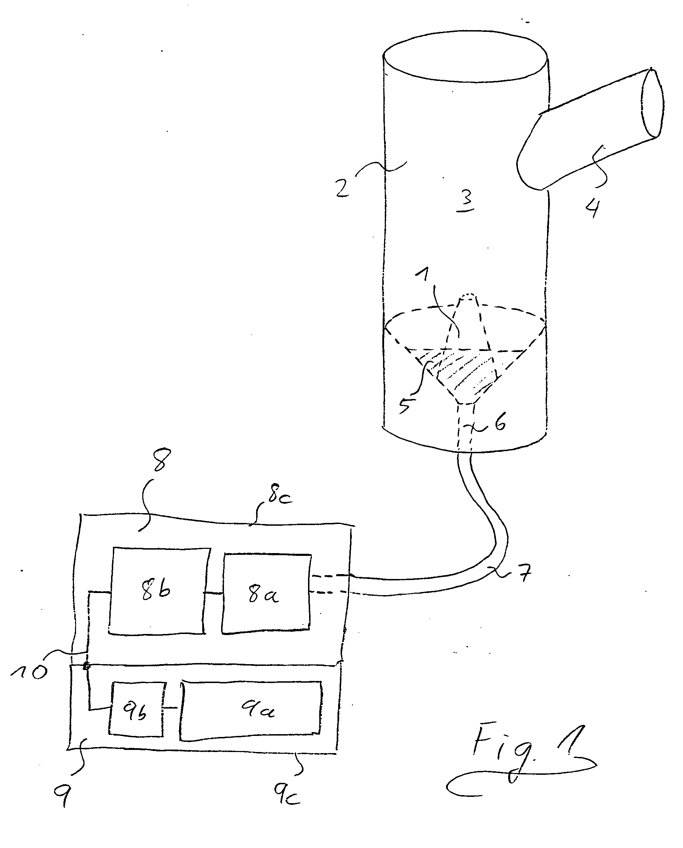

- Figur 1: zeigt ein Ausführungsbeispiel einer erfindungsgemäßen Inhalationstherapievorrichtung.

Das in Figur 1 gezeigte Ausführungsbeispiel einer erfindungsgemäßen Inhalationstherapievorrichtung umfasst eine Verneblerdüse 1, die in einem Verneblergehäuse 2 angeordnet ist, das einen Verneblungsraum 3 umschließt. Über ein Mundstück 4 atmet ein Patient ein von der Veneblerdüse 1 erzeugtes Aerosol aus dem Verneblungsraum 3 ein. Für die Erzeugung des Aerosols wird der Verneblerdüse 1 eine Flüssigkeit 5, die vorteilhafterweises in dem Verneblergehäuse 2 bevorratet wird, und Druckluft 6 zugeführt.

Die Druckluft 6, die vorzugsweise über eine Schlauchleitung 7 oder ähnliches zu der Verneblerdüse 1 herangeführt wird, wird von einem Kompressor 8 bereitgestellt. Dazu umfasst der Kompressor 8 typischerweise eine Drucklufterzeugungseinrichtung 8a, beispielsweise eine Kolben-, Membran- oder Zentrifugalpumpe, und eine elektromotorische Antriebseinrichtung 8b, vorzugsweise einen Elektromotor.

Der elektromotorischen Antriebseinrichtung 8b wird für den Betrieb elektrische Energie von einem elektrischen Energiespeicher 9 zugeführt, der neben einem Speicherelement 9a für elektrische Energie erfindungsgemäß eine Einrichtung 9b für die Gewährleistung einer konstanten Ausgangsspannung aufweist. Damit wird dem elektromotorischen Antrieb 8b des Kompressors 8 an der Zuführungsleitung 10 die elektrische Energie bei einer konstanten Spannung zugeführt. Während der gesamten Entladezeit des Speicherlements 9a wird eine konstante Ausgabespannung und damit eine konstante Leistung abgegeben. Eine Anpassung des elektrischen Energiespeichers an die angeschlossene Last, beispielsweise durch Anpassung der Anzahl der Speicherelemente 9a, ist bei der erfindungsgemäßen Gestaltung nicht erforderlich.

In einer vorteilhaften Ausgestaltung ist die Konstanthalteeinrichtung 9b ein DC/DC-Wandler, dem eingangsseitig Spannungen in einem vorgegebenen Bereich zugeführt werden können und der ausgangsseitig eine konstante Ausgangsspannung abgibt.

In einer weiteren vorteilhaften Ausgestaltung ist die Konstanthalteeinrichtung 9b, wie in Figur 1 gezeigt, in einem Gehäuse 9c des elektrischen Energiespeichers 9 untergebracht. Dadurch entsteht eine kompakte Einheit, die einfach zu handhaben ist. Vorzugsweise ist das Gehäuse 9c des elektrischen Energiespeichers 9 an die Form und Größe des Gehäuses des Kompressors 8 angepasst, was in Figur 1 ebenfalls dargestellt ist.

## Patentansprüche

1. Inhalationstherapievorrichtung mit einer Verneblerdüse (1), der für die Vernebelung einer Flüssigkeit (5) Druckluft (6) zuführbar ist, einem Kompressor (8), der einen elektromotorischen Antrieb (8b) aufweist, für die Erzeugung von der Verneblerdüse (1) zugeführter Druckluft (6), und einem elektrischen Energiespeicher (9) für die Bereitstellung von elektrischer Energie für den elektromotorischen Antrieb (8b) des Kompressors (8), wobei der elektrische Energiespeicher (9) eine Konstanthalteeinrichtung (9b) umfasst, die eine konstante Ausgangsspannung des Energiespeichers (9) gewährleistet.

2. Inhalationstherapievorrichtung nach Anspruch 1, **dadurch gekennzeichnet, dass** die Konstanthalteeinrichtung (9b) ein DC/DC-Wandler ist.

3. Inhalationstherapievorrichtung nach Anspruch 1 oder 2, **dadurch gekennzeichnet, dass** der elektrische Energiespeicher (9) ein Gehäuse (9c) aufweist und die Konstanthalteeinrichtung (9b) in dem Gehäuse (9c) des elektrischen Energierspeichers (9) angeordnet ist.

4. Inhalationstherapievorrichtung nach Anspruch 3, **dadurch gekennzeichnet, dass** der Kompressor (8) ein Gehäuse (8c) aufweist und dass das Gehäuse (9c) des elektrischen Energiespeichers (9) in Form und Größe an das Gehäuse (8c) des Kompressors (8) angepasst ist.
